(19) Europäisches Patentamt
European Patent Office
Office européen des brevets

(11) Publication number: **0 232 153 B1**

(12) **EUROPEAN PATENT SPECIFICATION**

(45) Date of publication of patent specification: **10.08.94**

(51) Int. Cl.5: **C11D 1/86**, C11D 1/94, C11D 1/83, A61K 7/08

(21) Application number: **87300879.1**

(22) Date of filing: **02.02.87**

The file contains technical information submitted after the application was filed and not included in this specification

(54) **Detergent compositions.**

(30) Priority: **03.02.86 GB 8602589**

(43) Date of publication of application:
**12.08.87 Bulletin 87/33**

(45) Publication of the grant of the patent:
**10.08.94 Bulletin 94/32**

(84) Designated Contracting States:
**CH DE ES FR GB IT LI NL SE**

(56) References cited:
**EP-A- 0 036 625**
**EP-A- 0 155 737**
**EP-A- 0 181 212**
**US-A- 3 928 249**
**US-A- 4 554 098**

(73) Proprietor: **UNILEVER PLC**
**Unilever House**
**Blackfriars**
**P.O. Box 68**
**London EC4P 4BO (GB)**
(84) Designated Contracting States:
**GB**

(73) Proprietor: **UNILEVER N.V.**
**Weena 455**
**NL-3013 AL Rotterdam (NL)**
(84) Designated Contracting States:
**CH DE ES FR IT LI NL SE**

(72) Inventor: **Naik, Appaya Raghunath**
**8 Kilmalcolm Close**
**Wexford Road**
**Oxton Burkenhead Merseyside (GB)**
Inventor: **Coxon, Andrew Charles**
**29 Gotham Road**
**Dibbens Hey**
**Bebington Wirral L63 9NG (GB)**

(74) Representative: **Ford, Michael Frederick et al**
**MEWBURN ELLIS**
**York House**
**23 Kingsway**
**London WC2B 6HP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 232 153 B1

## Description

The present invention relates to liquid detergent compositions suitable for use especially, but not exclusively, in fabric washing, shampoos, and above all, in manual dishwashing operations in both hard and soft water.

The term "dishes" as used herein means any utensils involved in food preparation or consumption which may be required to be washed to free them from food particles and other food residues, greases, proteins, starches, gums, dyes, oils and burnt organic residues.

Light-duty liquid detergent compositions such as are suitable for use in washing dishes are well-known. Many of the formulations in commercial use at the present time are based on a sulphonate-type anionic detergent, especially on alkyl benzene sulphonate, in conjunction with an alkyl polyethoxy sulphate (alkyl ether sulphate). The sulphonate-type detergent generally predominates.

The use of conventional dishwashing liquids based on alkyl benzene sulphonate/alkyl ether sulphate or on alkyl sulphate/alkyl ether sulphate is seen to have deleterious influence on hand conditions of users. Hence mildness in washing-up liquid is considered a desirable quality, and many specially formulated products on the market make claims for it.

Protein denaturation by surfactants is considered to be one of the major causes of skin irritation and skin roughness induced by surfactants (G. Imokawa et al JAOCS 52 484-489 Dec. 1975). The degree of surfactant denaturation of protein depends on the type of surfactants and their concentration.

At present the dishwashing formulations that are on the market which are less interactive with protein and hence considered to be milder are those based on a combination of ether sulphates and amine oxides. See for example US-A-3 928 249 (Procter and Gamble). In place of amine oxides, betaines can also be used. See for example US-A-4 554 098. However, such formulations - particularly with amine oxides - are expensive not only because the active ingredients are expensive but also because they require a large amount of expensive hydrotropes such as xylenesulphonate and/or ethanol to produce liquids which are stable and of accepted viscosity. Therefore there is a need for the development of more cost-effective mild dishwashing formulations, which are based on relatively less expensive detergent raw materials and which also require lesser amounts of expensive hydrotropes.

EP-A-36625 discloses compositions intended to give mildness and performance. One Example contains alkyl sulphate, alkyl ether sulphate, betaine and an ethoxylated alkyl phenol, together with isopropanol in aqueous solution.

The present invention is based on the realisation that cost-effective mild liquid dishwashing formulations with adequate performance can be obtained by careful choice of the active ingredients and their preparations in particular by restricting the main anionic detergent active present and including also selected amounts of alkyl ether sulphate, lather boosters and non-ionic detergent active material.

According to the present invention there is provided a stable detergent composition in liquid or gel form containing from 10 to 80% by weight of active detergent mixture and water, the active detergent mixture comprising four components as follows:

(a) anionic detergent active other than alkyl ether sulphate and which is present in an amount in the range 10 to 60% by weight of the active detergent mixture;

(b) alkyl ether sulphate containing one or more ethylene oxide residues per molecule, the weight ratio of components (a) and (b) being in the range 2:1 to 1:10, the proportion of component (b) with a single ethylene oxide residue per molecule being not substantially greater than the proportion with the most frequently occurring higher number of ethylene oxide residues per molecule;

(c) lather booster selected from the group consisting of monoalkanolamides, dialkanolamides, betaines, amine oxides and mixtures thereof in a total amount in the range 5 to 30% by weight of the active detergent mixture, the amount of monoalkanolamide not exceeding 20% by weight, the amount of amine oxide not exceeding 10% by weight, and the total amount of monoalkanolamide, dialkanolamide and betaine plus $1\frac{1}{2}$ times the amount of amine oxide being at least 7.5% by weight of the active detergent mixture;

(d) nonionic detergent active material in an amount in the range 5 to 35% by weight of the active detergent mixture, which nonionic detergent active is selected from the group consisting of polyethoxylated aliphatic alcohols of the general formula:

$$R - (OCH_2CH_2)m - OH$$

wherein R is straight or branched alkyl having from 8 to 16 carbon atoms, and the average degree of ethoxylation m is from 5 to 14, and

polyethoxylated alkanolamides of the general formula

$$R - \underset{\displaystyle\overset{|}{Y}}{CON}(R_8)(OCH_2CH_2O)_pH$$

wherein R is a straight or branched alkyl having from 7 to 18 carbon atoms,
$R_8$ is an ethyleneoxy or propyleneoxy group
Y is hydrogen or $-R_8(CH_2CH_2O)_qH$
p is 1 or more and q is 0, 1 or more;
with a further limitation on the amount of anionic detergent active (a) which is explained below and the proviso that primary alkane sulphonate is absent and the weight of any hydrotrope in the composition is not more than 12% calculated on the weight of the active detergent mixture.

For reasons of cost effectiveness, and availability of materials, at least a major proportion of the component (a) is preferably selected from secondary alkane sulphonate, alkylbenzene sulphonate, primary alkyl sulphate, fatty acyl ester sulphonate, dialkylsulphosuccinate and alpha-olefin sulphonate.

The anionic detergent active (a) must not exceed a maximum amount which is dependent on the nature of this active. For instance if secondary alkane sulphonate is substantially the only active (a) then the amount of it should not substantially exceed 50% by weight of the weight of the active detergent mixture.

Maxima for other detergent actives, when substantially alone, are:

| | |
|---|---|
| alkyl aryl sulphonate | 30% |
| alkyl sulphate | 40% |
| fatty acyl ester sulphonate | 50% |
| dialkylsulphosuccinate | 60% |
| alpha olefin sulphonate | 60% |

If anionic detergent active (a) is a mixture, then the maximum for the mixture is calculated as the total of the above maxima scaled in proportion to the amount of each active in the mixture.

The maximum amount of anionic detergent active (a) is expressed by specifying that the active detergent mixture has maximum contents:

u of alkyl aryl sulphonate
w of alkyl sulphate
x in total of secondary alkane sulphonate and fatty acyl ester sulphonate
y in total of dialkylsulphosuccinate amd alpha-olefin sulphonate, and
z in total of any other anionic active present - except of course for the alkyl ether sulphate which is component (b)-where u, w, x, y and z are all percentages by weight of the detergent active mixture and are given by the relationship:

$$\frac{u}{30} + \frac{w}{40} + \frac{x}{50} + \frac{y}{60} + \frac{z}{60} \leq 1$$

As already stated, the anionic detergent active (a) is at least 10% by weight of the active detergent mixture. Preferably it is at least 15% or 20%. It does not provide more than 60% of the active detergent mixture.

Preferred maxima for anionic detergent actives are lower:

| | |
|---|---|
| alkyl aryl sulphonate | 22.5% |
| alkyl sulphate | 30% |
| secondary alkane sulphonate | 37.5% |
| fatty acyl ester sulphonate | 37.5% |
| dialkylsulphosuccinate | 50% |
| alpha-olefin sulphonate | 50% |
| any other anionic detergent | active 50% or 40% |

Compositions of the invention can give performances in detergency tests as good as those of conventional compositions containing alkyl benzene sulphonate, but are considerably milder.

A significant form of this invention employs secondary alkane sulphonate as anionic detergent active (a) and monoethanolamide or diethanolamide as the lather booster (c), the active detergent mixture having in addition to the secondary alkane sulphonate and alkyl sulphate introduced with the alkyl ether sulphate a maximum content of 5% in total of alkyl sulphate and alkylaryl sulphonate material and preferably being substantially free of such additional sulphate and sulphonate material.

It is particularly surprising that the harsh actives alkylarylsulphonate and primary alkyl sulphate can be tolerated at levels as high as 30 and 40% by weight of the detergent active mixture. A significant aspect of this invention therefore resides in a four component composition as specified above in which primary alkyl sulphate, alkylbenzene sulphonate or a mixture of the two constitutes at least 10% of the active detergent mixture and at least a majority of the component (a).

In this invention it may be preferred that the total amount of alkyl benzene sulphonate and primary alkyl sulphate is no greater than the total of components (c) and (d). The total of these anionic detergents plus any secondary alkane sulphonate and fatty acyl ester sulphonate may be no greater than the total of (c) and (d). The total amount of component (a) is preferably no greater than $1\frac{1}{4}$ times the total of (c) and (d). In some forms of the invention the total amount of (a) is no greater than the total of (c) and (d).

Although in principle the concentration of the active detergent mixture may be as high as desired in the range 10 to 80%, provided that a stable liquid or gel product can be obtained, the range of 10 to 60% by weight is preferred, and aqueous liquid compositions with an active detergent mixture in the range of 10 to 40% by weight are of especial interest.

Six detergent actives have been mentioned above as possibilities for component (a). Of these, secondary alkane sulphonate may be preferred. It is generally produced by a free radical reaction, either a sulphochlorination reaction (Reed reaction) of a paraffin

$$RH + SO_2 + Cl_2 \rightarrow RSO_2Cl + HCl$$

followed by hydrolysis and neutralisation, or by a sulphoxidation reaction

$$RH + SO_2 + \tfrac{1}{2}O_2 \rightarrow RSO_3H$$

followed by neutralisation.

The secondary alkane sulphonate component (a) will normally be a mixture of materials of different alkyl chain lengths, of the formula:

$$R_1R_2CHSO_3X$$

where $R_1$ and $R_2$ which may be the same or different are each a straight or branched chain alkyl group having at least one carbon atom, the alkyl chain length (i.e. total number of carbon atoms of $R_1$ and $R_2$ plus 1) preferably being in the range 13 to 18, and X is a solubilising cation. An example of such material is SAS 60 of Hoechst (SAS is a Registered Trade Mark) which is produced by a sulphoxidation process.

A suitable secondary alkane sulphonate produced by a sulphochlorination process is Mersolat (trade mark) of Bayer.

Alkylbenzene sulphonate for use in component (a) is preferably derived from an alkylbenzene with a $C_8$ to $C_{16}$ primary or secondary alkyl group. This may in particular be $C_8$ to $C_{13}$ alkyl. Suitable alkylbenzene sulphonates are Dobs 102 of Shell and Marlon A of Hüls. (Dobs and Marlon are Registered Trade Marks)

Primary alkyl sulphate (primary alcohol sulphate) for use in component (a) is preferably of the formula

$$R\text{-}OSO_3X$$

where R is a $C_8$ to $C_{16}$ primary alkyl group and X is a solubilising cation. Suitable is Dobanol 23A of Shell in which R is predominantly $C_{12}$ and $C_{13}$. As will be explained in more detail below alkyl sulphate is a constituent of alkyl ether sulphates which provide component (b). Dobanol is a Registered Trade Mark.

Suitable detergent-active dialkyl sulphosuccinates are compounds of the formula

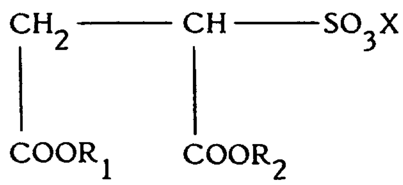

wherein each of $R_1$ and $R_2$, which may be the same or different, represents a straight-chain or branched-chain alkyl group having 3 to 12 carbon atoms, preferably from 4 to 10 carbon atoms and more preferably from 6 to 8 carbon atoms, and X represents a solubilising cation.

The alkyl groups $R_1$ and $R_2$ are preferably straight-chain or (in mixtures) predominantly straight-chain.

Among dialkyl sulphosuccinates that may advantageously be used in the composition of the invention are the $C_6/C_8$ unsymmetrical materials described and claimed in GB-A-2 105 325 (Unilever); the dioctyl sulphosuccinate/dihexyl sulphosuccinate mixtures described and claimed in GB-A-2 104 913 (Unilever); and the mixtures of symmetrical and unsymmetrical dialkyl sulphosuccinates described and claimed in GB-A-2 108 520 (Unilever).

Appropriate alpha-olefin sulphonates will generally be in the range having from twelve to sixteen carbon atoms.

An example of such a material is Liporan 440, a $C_{14}$ alpha-olefin sulphonate from Lion Corporation, Japan. Liporan is a Registered Trade Mark.

Appropriate fatty acid ester sulphonates are of formula

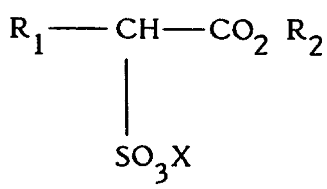

where

$R_1$ is straight or branched $C_8$ to $C_{16}$ alkyl

$R_2$ is straight or branched $C_1$ to $C_4$ alkyl

and

X is a solubilising cation.

$R_1$ is preferably $C_{10}$ to $C_{12}$

and

$R_2$ is preferably butyl.

Component (a) of the active detergent mixture may also include one or more other detergent actives used in liquid compositions, for example alkyl glyceryl ether sulphonates, or alkyl sarcosinates. The amount is preferably less than half of component (a), more preferably not more than a quarter of it.

The second essential component (b) of the active detergent mixture is alkyl ether sulphate (sometimes called alcohol ether sulphate or alkyl polyethoxy sulphate) having at least one ethylene oxide residue per molecule. This will normally be provided by incorporating into the composition an alkyl ether sulphate which is a mixture of materials of the general formula:

$$R - (OCH_2CH_2)_n - OSO_3X$$

wherein R is a $C_{10}$ to $C_{18}$ primary or secondary alkyl group, X is a solubilising cation, and $\underline{n}$, the average degree of ethoxylation, is at least 1.5, preferably from 2 to 12, more preferably 3 to 8. Particularly preferred values of $\underline{n}$ are 3, 4 and 5. $R_3$ is preferably a $C_{10}$ to $C_{16}$ alkyl group. In any given alkyl ether sulphate, a range of differently ethoxylated materials, and some unethoxylated material, will be present and the value of $\underline{n}$ represents an average. The unethoxylated material is, of course, alkyl sulphate and this contributes to component (a).

The amount of alkyl sulphate in any alkyl ether sulphate will depend on the average degree of ethoxylation n. When n is 3, alkyl sulphate typically constitutes 15 to 20% of the mixture, and less than this when n is 4 or more. When the proportion of alkyl sulphate is low, it may prove convenient to ignore it. Nevertheless it contributes to component (a).

When the average degree of ethoxylation is 2, alkyl sulphate typically constitutes 30% of the mixture provided as "alkyl ether sulphate". Such a mixture can provide both component (b) and component (a), with the latter then consisting entirely of alkyl sulphate.

We have found that it is not feasible to use only an alkyl ether sulphate with an average degree of ethoxylation below 1.5. Unless the alkyl sulphate content of the ether sulphate is providing much or the whole of component (a), it is preferred that the alkyl ether sulphate is provided by material with an average of at least 2 or 2.5 ethylene oxide residues per molecule.

An alkyl ether sulphate usually contains molecules with differing numbers of ethylene oxide residues in a statistical distribution. In such an alkyl ether sulphate, provided the average degree of ethoxylation is sufficiently great, the proportion of molecules with a single ethylene oxide residue will be not substantially greater than, or less than, the proportion with two ethylene oxide residues and the proportion with the most frequently encountered number of ethylene oxide residues (if this is more than two) as this invention requires.

Preferred alkyl ether sulphates of the component (b), excluding alkyl sulphate, are mixtures of compounds of the above formula

$R-(OCH_2CH_2)_n-OSO_3X$

in which n is any positive integer.

It is preferred, for this invention, to use primary alkyl ether sulphates containing less than 20% by weight of material with alkyl groups of $C_{14}$ and above material, more preferably substantially $C_{12}$ and $C_{13}$ material only. Such material preferably has an average degree of ethoxylation of 2 to 6, more preferably 3 to 5.

Examples of preferred alkyl ether sulphates for use in the present invention are Dobanol (Registered Trade Mark) 23-3 from Shell in which the degree of ethoxylation (n) is 3 and the equivalent material in which the degree of ethoxylation is 4. These materials are based on $C_{12}$-$C_{13}$ (50% of each) primary alcohol (about 75% straight chain, 25% 2-methyl branched). Another preferred material is an alkyl ether sulphate based on Lial (Registered Trade Mark) 123 from Chimica Augusta, which is a branched chain primary alcohol with a degree of ethoxylation of 3 to 4.

A suitable example of a secondary alcohol ether sulphate is a material derived from an alcohol such as Tergitol 15/S/3 (Registered Trade Mark) of Union Carbide (this material itself is not at present available). The conventional process of manufacture of secondary alkyl ether sulphates is such that there is only a very small quantity of alkyl sulphate in the product.

Component (b) provides at least 12% of the active detergent mixture, preferably it provides at least 15 or 20%. It preferably provides not more than 60 or 65% of the active detergent mixture.

The solubilising cations of the anionic detergent actives of components (a) and (b), denoted as X in formulae above, may be any which provide the desired solubility of the anionic material. Monovalent cations such as alkali metal ions, ammonium and substituted ammonium are typical. Divalent ions giving adequate solubility may be used, and especially magnesium ions may be present to improve soft water performance and can be incorporated as magnesium salt of the anionic actives or as inorganic magnesium salts, or in the hydrotrope system.

Component (c) of the active detergent mixture of the composition of the invention is preferably one or more mono- or dialkanolamides, preferably $C_8$ to $C_{18}$, more preferably $C_{10}$-$C_{18}$ carboxylic acid mono- or di-($C_2$-$C_3$) alkanolamides. These have the general formulae

$R_4$- CO - $NHR_5$ and $R_4$ - CO - $N(R_5)_2$ respectively

wherein $R_4$ is a $C_7$-$C_{17}$ aliphatic group, preferably straight-chain and preferably saturated, and $R_5$ is a hydroxyethyl or hydroxypropyl group. $R_5$ is preferably a 2-hydroxyethyl group.

Materials of this type are generally made from fatty acids of natural origin and contain a range of molecules having $R_4$ groups of different chain lengths; for example, coconut ethanolamides consist predominantly of $C_{12}$ and $C_{14}$ material, with varying amounts of $C_8$, $C_{10}$ and $C_{16}$ material. Preferred are ethanolamides derived from so-called middle cut coconut fatty acid, most preferably from lauric acid.

Monoalkanolamide may be used alone (in an amount in the range 7.5 to 20% of the active detergent mixture) or dialkanolamide may be used alone (in an amount in the range 7.5 to 30% of the active detergent mixture). It is preferred to use a mixture of mono- and dialkanolamide, most preferably in a weight ratio of 2:1 to 1:2.

The other possibilities for component (c) are betaines and amine oxides. It is preferred to avoid using substantial amounts of these, especially amine oxides, for the sake of economy and consequent cost effectiveness. Preferably then the amount of amine oxide is not more than 10% by weight of the active detergent mixture. Preferably the amount of betaine is not more than 20% by weight of this mixture. The total amount of amine oxide and betaine is preferably not more than 20% and more preferably not more than 15% or 10% by weight of the active detergent mixture.

It may be convenient to use amine oxide and/or betaine at such low levels, as part or even all of component (c).

In important forms of the invention, however, amine oxide is excluded.

Suitable betaines include simple betaines of formula

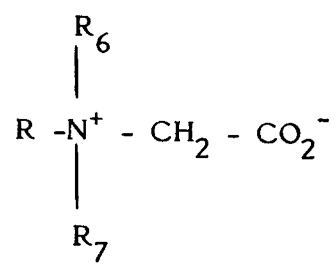

and amido betaines of formula:

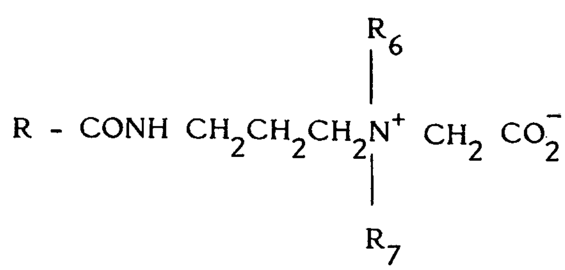

In both formulae R is a $C_7$ to $C_{16}$ straight or branched alkyl group. It may be a lauryl group or a middle cut coconut alkyl group. $R_6$ and $R_7$ are each $C_1$ to $C_3$ alkyl or $C_1$ to $C_3$ hydroxyalkyl. Examples of sulphobetaines have the above formulae with $-CH_2CO_2^-$ replaced by

$-(CH_2)_3SO_3^-$ or

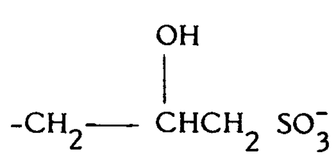

A suitable simple betaine is Empigen BB from Albright and Wilson (Empigen is a Registered Trade Mark). It has the formula quoted above in which R is $C_{12}$ to $C_{14}$ alkyl, derived from coconut, and $R_6$ and $R_7$ are both methyl.

Suitable amine oxides have the formula

$R\ R_6\ R_7\ N \rightarrow O$

wherein R is a straight or branched chain $C_8$ to $C_{18}$ alkyl group and $R_6$ and $R_7$ are each $C_1$ to $C_3$ alkyl, or

$C_1$ to $C_3$ hydroxyalkyl. A suitable amine oxide is Empigen OB from Albright and Wilson. In it R is $C_{12}$ to $C_{14}$ alkyl, derived from coconut and $R_6$ and $R_7$ are both methyl.

Component (c) preferably constitutes at least 10% e.g. 12% to 25%, by weight of the active detergent mixture.

Component (d) is one or more non-ionic detergent active materials. The alkanolamides, betaines and amine oxides of component (c) do not form part of component (d).

It is desirable that component (d) should have an HLB value in the range from 12.0 to 16.0.

Component (d) is preferably a polyethoxylated aliphatic alcohol having an alkyl chain length of from $C_8$ to $C_{16}$, and an average degree of ethoxylation of from 4 to 14. Suitable nonionic detergents include short-chain high-foaming ethoxylated alcohols of the general formula

$R - (OCH_2CH_2)_m - OH$

wherein R is an alkyl group, preferably straight-chain, having from 8 to 16 and yet more preferably 9 to 11, carbon atoms, and the average degree of ethoxylation m is from 5 to 14, more preferably 6 to 12. An especially preferred nonionic detergent is Dobanol 91-8 from Shell, in which R is $C_9$-$C_{11}$ (predominantly straight-chain) and m is 8.

Alternative suitable materials are those in which R is a secondary alkyl having from 8 to 16, preferably 11-15, carbon atoms and m is from 5 to 14 preferably 6-12. An example is Tergitol 15/S/12 of Union Carbide (not available at present) or the material of the Softanol A series (from Japan Catalytic). Tergitol and Softanol are Registered Trade Marks.

Preferably the polyethoxylated alcohol mixture is stripped, to reduce odour imparted to the composition.

As stated earlier, an alternative possibility for the component (d) is an ethoxylated alkanolamide of the general formula

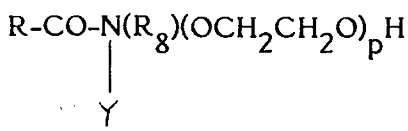

wherein R is a straight or branched alkyl having from 7 to 18 carbon atoms,

$R_8$ is an ethyleneoxy or propyleneoxy group

Y is hydrogen or $-R_8(CH_2CH_2O)_qH$

p is 1 or more and q is 0, 1 or more.

R may be lauryl or coconut alkyl. Examples of ethoxylated alkanolamide are Amidox L5 and Amidox C5 from Stepan Chemical Company. Amidox is a Registered Trade Mark.

Component (d) preferably constitutes at least 10% by weight of the active detergent mixture e.g. 12 to 25%. The total of components (c) and (d) together may constitute at least 25% by weight of the active detergent mixture.

As well as the active detergent mixture and water, the liquid detergent compositions of the invention will generally need to contain one or more hydrotropes. Hydrotropes are materials present in a formulation to control solubility, viscosity, clarity and stability but which themselves make no active contribution to the performance of the product. Examples of hydrotropes include lower aliphatic alcohols, especially ethanol; urea; lower alkylbenzene sulphonates such as sodium, toluene and xylene sulphonates and combinations of these. Preferred are alcohol, urea, and xylene sulphonate. Hydrotropes are expensive and take up room in a formulation without contributing to its performance, and it is therefore desirable to use as small quantities of them as possible.

For example, the use of amine oxides as mentioned above requires a large amount of alcohol as hydrotrope. For this reason and because of expense, it is preferred to avoid the use of a substantial amount of any tertiary amine oxide in the present invention.

In this invention the weight of hydrotrope in the composition is not more than 12% of the weight of the active detergent mixture.

The compositions of the invention may also contain the usual minor ingredients such as perfume, colour, preservatives and germicides.

The stable liquid detergent compositions of the invention may be used for all normal detergent purposes especially where foaming is advantageous, for example, fabric washing products, general purpose domestic and industrial cleaning compositions, carpet shampoos, car wash products, personal washing

products, shampoos, foam bath products, and above all, manual dishwashing.

The invention is further illustrated by the following non-limiting Examples.

EXAMPLE 1

The foaming performances of various aqueous formulations were compared using a plate washing test. In the test, plates soiled with a standard starch/fat/fatty acid mixture were washed in a standard manner with 5 litres of test solution (total concentration of the product 1 g/litre in 24°H (French hardness) water at 45°C in a bowl, until a third of the surface of the solution in the bowl was covered with foam. The number of plates washed before this arbitrary end-point was reached was taken as an indicator of dishwashing and foaming performance.

In Table 1 below, the content of each component is given as percentage by weight of the whole composition. For each composition the total active detergent concentration (including the ethanolamide materials) was kept constant at 40%.

In Table 2 below the content of the same compositions is expressed as percentages by weight of the detergent active mixture, with the primary alkyl sulphate (from the alkyl ether sulphate) shown separately.

The foaming performance, cloud point and viscosity of various compositions of the invention (I-VI), a comparable composition containing alkyl benzene sulphonate (A), a comparable composition containing only secondary alkane sulphonate and alkyl ether sulphate (B), and a comparable composition containing no sulphonate (C) were obtained.

The lauric diethanolamide used was a commercial narrow-cut coconut diethanolamide, referred to as LDEA, and the cocomonoethanolamide was Empilan CME of Albright & Wilson.

The formulations and results are set out in Table 1 below and repeated in Table 2 expressed as percentages by weight of the active detergent mixtures.

TABLE 1

| Composition No. | I | II | III | IV | V | VI | A | B | C |
|---|---|---|---|---|---|---|---|---|---|
| Component | Percentages by weight of the formulation | | | | | | | | |
| SAS60 | 12 | 12 | 12 | 13 | 13 | 13 | - | 32 | - |
| Marlon A | - | - | - | - | - | - | 12 | - | - |
| Dobanol 23-3A | 18 | 18 | - | 13 | - | 13 | 18 | 8 | 24 |
| Dobanol 23-4S | - | - | 18 | - | 13 | - | - | - | - |
| CEA | 4 | 4 | 4 | 4 | 4 | - | - | - | 4 |
| LDEA | - | - | - | 4 | 4 | 8 | 4 | - | 4 |
| Dobanol 91-8EO | 6 | 6 | 6 | 6 | 6 | 6 | 6 | - | 8 |
| Urea | 4 | - | 4 | 4 | 4 | 4 | 5 | 3 | - |
| IMS | - | 3 | - | - | - | - | 4 | 3 | 9 |
| $MgSO_4 7H_2O$ | 2.5 | 2.5 | 2.5 | 2.5 | 2.5 | 2.5 | 2.5 | - | 2 |
| Cloud pt. (°C) | +2 | -5 | +1 | -4 | -4 | -4 | -4 | -1 | +13 |
| Viscosity (mPa.s) | 208 | 168 | 320 | 224 | 320 | 272 | 196 | 280 | 192 |
| Plates Test | 27 | 27 | 28 | 28 | 28 | 26 | 30 | 27 | 21 |

| | | |
|---|---|---|
| SAS* 60 | - $C_{13-18}$ secondary alkane sulphonate | - Hoechst |
| Marlon* A | - $C_{10-13}$ alkyl benzene sulphonate | - Hüls |
| Dobanol* 23-3A | - $C_{12-13}$ alcohol 3EO(ethylene oxide) sulphate | - Shell |
| Dobanol* 23-4S | - $C_{12-13}$ alcohol 4EO sulphate | - Shell |
| CEA | - coco monoethanolamide (Empilan* CME) | - Albright & Wilson |
| LDEA | - lauric diethanolamide | |
| Dobanol* 91-8EO | - $C_{9-11}$ alcohol 8EO | - Shell |
| IMS | - industrial methylated spirit | |
| * | - trade mark | |

TABLE 2

| Composition No. | I | II | III | IV | V | VI | A | B | C |
|---|---|---|---|---|---|---|---|---|---|
| Component | Percentages by weight of active detergent mixture | | | | | | | | |
| SAS60 | 30 | 30 | 30 | 32.5 | 32.5 | 32.5 | - | 80 | - |
| Marlon A | - | - | - | - | - | - | 30 | - | - |
| alkyl sulphate | 7 | 7 | 4.5 | 5 | 3.5 | 5 | 7 | 3 | 9.5 |
| alkyl ether sulphate excluding alkyl sulphate | 38 | 38 | 40.5 | 27.5 | 29 | 27.5 | 38 | 17 | 50.5 |
| CEA | 10 | 10 | 10 | 10 | 10 | - | - | - | 10 |
| LDEA | - | - | - | 10 | 10 | 20 | 10 | - | 10 |
| Dobanol 91-8EO | 15 | 15 | 15 | 15 | 15 | 15 | 15 | - | 20 |
| Hydrotrope | 10 | 7.5 | 10 | 10 | 10 | 10 | 22.5 | 15 | 22.5 |
| Cloud pt. (°C) | +2 | -5 | +1 | -4 | -4 | -4 | -4 | -1 | +13 |
| Viscosity (mPa.s) | 208 | 168 | 320 | 224 | 320 | 272 | 196 | 280 | 192 |
| Plates Test | 27 | 27 | 28 | 28 | 28 | 26 | 30 | 27 | 21 |

SAS* 60 - $C_{13-18}$ secondary alkane sulphonate - Hoechst
Marlon* A - $C_{10-13}$ alkyl benzene sulphonate - Hüls
Dobanol* 23-3A - $C_{12-13}$ alcohol 3EO(ethylene oxide) sulphate - Shell
Dobanol* 23-4S - $C_{12-13}$ alcohol 4EO sulphate - Shell
CEA - coco monoethanolamide (Empilan* CME) - Albright & Wilson
LDEA - lauric diethanolamide
Dobanol* 91-8EO - $C_{9-11}$ alcohol 8EO - Shell
IMS - industrial methylated spirit
* - trade mark

The compositions (I-VI) of the invention had acceptable hydrotrope levels and viscosities and foaming performances only slightly inferior to composition A, as good as that of composition B and much better than that of composition C.

Mildness of some of the above formulations was estimated using a test of protein denaturation potency.

Several in vitro and in vivo methods for evaluating protein denaturation potency of surfactants and their mixtures have been reported (see K. Miyazawa et al Int J Cos Sci 6 33-46 1984 and the references cited therein). One such method is the study of interaction of skin acid phosphatase enzyme with detergents under realistic conditions reported by Prottey et al (Int J Cos Sci 6 263-273 1984).

Formulations II, IV, A, B, and C above were tested by Prottey's method using dilute solutions at active concentrations giving equal plates test performance. The results expressed qualitatively as a rank order of predicted mildness are as follows:

C > IV ≈ II > A ≈ B

C was significantly better than IV and II, which were close together. A and B gave results similar to each other and very much worse than IV and II.

EXAMPLE 2

Human volunteers were employed to compare the active detergent mixtures of compositions II, IV and V and a commercial product identified as relatively mild. The detergent active in this product was a 4:1 mixture of secondary alkane sulphonate and alkyl ether sulphate (average 3 ethylene oxide residues per molecule). Thus it was closely similar to composition B.

The compositions were prepared at an active detergent concentration of 25% by weight without hydrotropes. Each trial involved panellists washing each volar forearm with a different one of the above products 4 times daily for 4 days and 3 times on day 5. For each wash 0.5 g of product was dosed onto the wetted volar forearm and it was washed with a gloved hand for 60 seconds. The forearm was rinsed after each wash. The products were randomly allocated to the panellists. Each volar forearm was graded for erythema dryness before each wash and 2 hours after the final wash. Any site reaching an erythema score denoting moderate erythema or a dryness score denoting moderate to severe dryness was discontinued.

The results showed that for potential to induce irritation V was significantly better than II and IV which were significantly better than the commercial product (composition B). For potential to induce skin dryness V was better than II or IV which were significantly better than composition B These results are consistent with Example 1.

EXAMPLE 3

A number of formulations were tested for interaction with skin acid phosphatase, by Prottey's method, using solutions of equal dilution. The compositions and the percentage inhibition (i.e. 100% minus percentage activity remaining) are set out in Table 3 below, where the compositions are expressed as percentages by weight of the formulation and then repeated in terms of percentages by weight of active detergent mixtures. Water gave no inhibition at all, i.e. 100% of activity remained.

TABLE 3

| Composition no: | 3.1 | 3.2 | 3.3 | 3.4 | 3.5 | 3.6 | 3.7 | 3.8 | 3.9 | 3.10 | 3.11 |
|---|---|---|---|---|---|---|---|---|---|---|---|
| Component | Percentages by weight of the formulation | | | | | | | | | | |
| SAS60 | 20 | 16 | 16 | 12 | 6 | 12 | 12 | 16 | 12 | 18 | 12 |
| Dobs 102 | - | - | - | 6 | - | - | - | - | - | - | - |
| Dobanol 23A | - | - | - | - | 12 | - | 6 | - | - | - | - |
| Dobanol 23-4A | 20 | 16 | 16 | 10 | 10 | 14 | 10 | 16 | - | 10 | 12 |
| Dobanol 23-2.5A | - | - | - | - | - | - | - | - | 12 | - | - |
| CEA | - | 4 | - | 4 | 4 | - | 4 | - | 4 | 4 | 4 |
| LDEA | - | 4 | - | - | - | 12 | - | - | 4 | - | 4 |
| Dobanol 91-8EO | - | - | - | 8 | 8 | 2 | 8 | 8 | 8 | 8 | 8 |
| | Percentages by weight of active detergent mixture | | | | | | | | | | |
| SAS60 | 50 | 40 | 40 | 30 | 15 | 30 | 30 | 40 | 30 | 45 | 30 |
| Dobs 102 | - | - | - | 15 | - | - | - | - | - | - | - |
| alkyl sulphate | 5 | 4 | 4 | 2.5 | 32.5 | 3.5 | 17.5 | 4 | 5.5 | 2.5 | 3 |
| alkyl ether sulphate excluding alkyl sulphate | 45 | 36 | 36 | 22.5 | 22.5 | 31.5 | 22.5 | 36 | 24.5 | 22.5 | 27 |
| CEA | - | 10 | - | 10 | 10 | - | 10 | - | 10 | 10 | 10 |
| LDEA | - | 10 | - | - | - | 30 | - | - | 10 | - | 10 |
| Dobanol 91-8EO | - | - | - | 20 | 20 | 5 | 20 | 20 | 20 | 20 | 20 |
| % inhibition | 72 | 63 | 61 | 58 | 44 | 41 | 41 | 41 | 23 | 21 | 13 |

| | | |
|---|---|---|
| Dobs 102 | $C_{10}$-$C_{12}$ alkylbenzene sulphonate | -Shell |
| Dobanol 23A | $C_{12}$-$C_{13}$ primary alkyl sulphate | -Shell |
| Dobanol 23-4A | $C_{12}$-$C_{13}$ alcohol 4EO (ethyleneoxide)sulphate | -Shell |
| Dobanol 23-2.5A | $C_{12}$-$C_{13}$ alcohol 2.5EO sulphate | -Shell |

EXAMPLE 4

In this and subsequent examples mildness is assessed by measuring the inhibition of wheatgerm acid phosphatase (WGAP). This has been found to correlate well with the results obtained with human volunteers: for instance the inhibition of WGAP by compositions B, IV and V in a series of comparative experiments was 100%, 75% and 69% respectively.

It is notable that the commercial product (composition B) gave total inhibition of this enzyme. Even a 3:7 mixture of SAS 60: Dobanol 23-3A gave 91% inhibition.

A series of compositions was assessed in which the nature of component (a) was varied, and the proportions of components (a) and (b) were also varied. These compositions were also assessed for foaming performance by means of a modified Schlachter-Dierkes test based on the principle described in Fette und Seifen 1951, 53, 207. A 100ml aqueous solution of each material tested, having a concentration of 0.04% active detergent in 24°H water (French hardness) at 45°C was rapidly oscillated using a vertically oscillating perforated disc within a graduated cylinder. After the initial generation of foam, increments (0.2g) of soil (9.5 parts commercial cooking fat, 0.25 parts oleic acid, 0.25 parts stearic acid and 10 parts wheat starch in 120 parts water) were added at 15-second intervals (10 seconds' mild agitation and 5 seconds' rest) until the foam collapsed. The result was recorded as the number of soil increments (NSI score): a score difference of 6 or less is generally regarded as insignificant. Each result was typically the average of 3 or 4 runs.

The compositions and the results obtained are set out in Table 4 below. In this table the compositions are quoted, and then calculated figures are given for the amounts of alkyl sulphate and alkyl ether sulphate excluding alkyl sulphate.

As can be seen from Table 4, the compositions' foaming performance was satisfactory in that it was never worse than the control composition, effectively composition V, shown in the final column of Table 4. Inhibition was never the total inhibition encountered with composition B (i.e. the commercial product). It can be seen that levels of 25% alkyl benzene sulphonate with 3.5% alkyl sulphate, and a level of 37.5% alkyl sulphate give about 85% inhibition, which is intermediate between the commercial product (composition B) and composition V.

TABLE 4

| Composition no: | 4.1 | 4.2 | 4.3 | 4.4 | 4.5 | 4.6 | 4.7 | 4.8 | 4.9 | 4.10 | 4.11 | 4.12 | 4.13 | V |
|---|---|---|---|---|---|---|---|---|---|---|---|---|---|---|
| Component | Percentages by weight of active detergent mixture | | | | | | | | | | | | | |
| SAS 60 | 20 | 25 | 30 | 35 | | | | | | | | | | 32.5 |
| Dobs 102 | | | | | 20 | 25 | 30 | 35 | | | | | | |
| Dobanol 23A | | | | | | | | | 20 | 25 | 30 | 35 | | |
| Dobanol 23-2A | | | | | | | | | | | | | 60 | |
| Dobanol 23-4A | 40 | 35 | 30 | 25 | 40 | 35 | 30 | 25 | 40 | 35 | 30 | 25 | | 32.5 |
| LME | 10 | 10 | 10 | 10 | 10 | 10 | 10 | 10 | 10 | 10 | 10 | 10 | 10 | 10 |
| LDEA | 15 | 15 | 15 | 15 | 15 | 15 | 15 | 15 | 15 | 15 | 15 | 15 | 15 | 10 |
| Dobanol 91-8EO | 15 | 15 | 15 | 15 | 15 | 15 | 15 | 15 | 15 | 15 | 15 | 15 | 15 | 15 |
| alkyl sulphate | 4 | 3.5 | 3 | 2.5 | 4 | 3.5 | 3 | 2.5 | 24 | 28.5 | 33 | 37.5 | 18 | 3 |
| alkyl ether sulphate excluding alkyl sulphate | 36 | 31.5 | 27 | 22.5 | 36 | 31.5 | 27 | 22.5 | 36 | 31.5 | 27 | 22.5 | 42 | 29 |
| % Inhibition of WGAP | 54 | 64 | 68 | 75 | 74 | 83 | 89 | 95 | 58 | 69 | 77 | 82 | 65 | 72 |
| Foaming performance | 35 | 36 | 38 | 38 | 37 | 40 | 44 | 43 | 37 | 39 | 43 | 40 | 35 | 34 |

Dobanol 23-2A $C_{12-13}$ alcohol 2EO (ethyleneoxide)sulphate -Shell

LME lauric monothanolamide

EXAMPLE 5

A number of compositions were assessed for WGAP inhibition. These included a series which were each the same as composition V, except that the secondary alkane sulphonate was replaced with an equal

weight of another anionic detergent active. Some were also assessed for foaming performance by means of the modified Schlacter-Dierkes test referred to above. The compositions and the results obtained are set out in Table 5 below.

"Sulphosuccinate" denotes a statistical mixture (mole ratio 1:2:1) of di-n-octyl sulphosuccinate, n-hexyl n-octyl sulphosuccinate and di-n-hexyl sulphosuccinate (sodium salts), prepared from a 1:1 mixture of n-hexanol and n-octanol by the method described in Example 1 of GB 2 108 520 (Unilever).

"Butyl FAES" denotes the sulphonate of n-butyl laurate.

TABLE 5

| Composition no: | V | 5.1 | 5.2 | 5.3 | 5.4 | 5.5 | 5.6 | 5.7 | 5.8 |
|---|---|---|---|---|---|---|---|---|---|
| Component | Percentages by weight of active detergent mixture | | | | | | | | |
| SAS 60 | 32.5 | | | | | | | | |
| Dobs 102 | | 32.5 | | | | | | | |
| Dobanol 23A | | | 32.5 | | | | | | |
| Butyl FAES | | | | 32.5 | | | | | |
| Sulphosuccinate | | | | | 32.5 | | | 57 | 57 |
| $C_{14}$ alpha-olefin sulphonate | | | | | | 32.5 | | | |
| Dobanol 23-3A | | | | | | | | 28.5 | |
| Dobanol 23-4A | 32.5 | 32.5 | 32.5 | 32.5 | 32.5 | 32.5 | 75 | | 28.5 |
| CEA | 10 | 10 | 10 | 10 | 10 | 10 | 10 | | |
| LDEA | 10 | 10 | 10 | 10 | 10 | 10 | 10 | 14.5 | 14.5 |
| Dobanol 91-8EO | 15 | 15 | 15 | 15 | 15 | 15 | 15 | | |
| % inhibition of WGAP | 72 | 90 | 80 | 70 | 60 | 55 | 35 | 82 | 80 |
| Foaming performance | 34 | | | | 46.5 | | | 49 | 51 |

The mildness arising with the different actives is apparent. It can also be seen that the composition of this invention, with sulphosuccinate as component (a) was superior in mildness to the sulphosuccinate compositions which were 4:2:1 sulphosuccinate: alkyl ether sulphate: diethanolamide, yet was fairly close in foaming performance, and better than the composition with secondary alkane sulphonate (composition V of Example 1).

EXAMPLE 6

The foaming performance of some further compositions was assessed by means of the modified Schlacter-Dierkes test. The compositions and the results obtained are set out in Table 6 below.

TABLE 6

| Component | Percentages by weight of active detergent mixture | | | | |
|---|---|---|---|---|---|
| SAS 60 | 32.5 | 32.5 | | | 32.5 |
| Dobanol 23-A | | | 21 | 21 | 32.5 |
| Dobanol 23-4A | 32.5 | 32.5 | 42 | 42 | |
| Lauryl mono ethanolamide | 10 | 10 | 10.5 | 10.5 | 10 |
| LDEA | 10 | 10 | | | 10 |
| Empigen OB | | | 10.5 | | |
| Empigen BB | | | | 10.5 | |
| Dobanol 91-8EO | | | 16 | 16 | 15 |
| Amidox L5 | 15 | | | | |
| Amidox C5 | | 15 | | | |
| Performance | 41 | 37 | 45 | 50 | 36 |

Amidox L5 is lauric monoethanolamide 5EO (ethylene oxide)
Amidox C5 is coconut monoethanolamide 5EO
Both are supplied by Stepan Chemical Company.
Empigen OB is $C_{12}$ to $C_{14}$ alkyl dimethyl amine oxide.
Empigen BB is $C_{12}$ to $C_{14}$ alkyl dimethyl betaine.

## Claims

**1.** A stable detergent composition in liquid or gel form containing from 10 to 80% by weight of active detergent mixture and water, the active detergent mixture comprising four components as follows:

(a) anionic detergent active other than alkyl ether sulphate and which is present in an amount in the range 10 to 60% by weight of the active detergent mixture;

(b) alkyl ether sulphate containing one or more ethylene oxide residues per molecule, the weight ratio of components (a) and (b) being in the range 2:1 to 1:10, the proportion of component (b) with a single ethylene oxide residue per molecule being not substantially greater than the proportion with the most frequently occurring higher number of ethylene oxide residues per molecule;

(c) lather booster selected from the group consisting of monoalkanolamides, dialkanolamides, betaines, amine oxides and mixtures thereof in a total amount in the range 5 to 30% by weight of the active detergent mixture, the amount of monoalkanolamide not exceeding 20% by weight, the amount of amine oxide not exceeding 10% by weight, and the total amount of monoalkanolamide, dialkanolamide and betaine plus $1\frac{1}{2}$ times the amount of amine oxide being at least 7.5% by weight of the active detergent mixture;

(d) nonionic detergent active material in an amount in the range 5 to 35% by weight of the active detergent mixture, which nonionic detergent active is selected from the group consisting of polyethoxylated aliphatic alcohols of the general formula:

$$R - (OCH_2CH_2)_m - OH$$

wherein R is straight or branched alkyl having from 8 to 16 carbon atoms, and the average degree of ethoxylation m is from 5 to 14, and

polyethoxylated alkanolamides of the general formula

$$R - \underset{\displaystyle Y}{\underset{|}{CON}}(R_8)(OCH_2CH_2O)_pH$$

wherein R is a straight or branched alkyl having from 7 to 18 carbon atoms,

$R_8$ is an ethyleneoxy or propyleneoxy group

Y is hydrogen or $-R_8(CH_2CH_2O)_qH$

p is 1 or more and a is 0, 1 or more; the active detergent mixture having maximum contents u of alkylaryl sulphonate, w of alkyl sulphate, a maximum total content x of secondary alkane sulphonate and fatty acyl ester sulphonate, a maximum total content y of dialkylsulphosuccinate and alpha-olefin sulphonate and a maximum total content z of any other anionic detergent active except said alkyl ether sulphate,

where u, w, x, y and z are all percentages by weight of the detergent active mixture and are given by

$$\frac{u}{30} + \frac{w}{40} + \frac{x}{50} + \frac{y}{60} + \frac{z}{60} \leqq 1$$

with the proviso that primary alkane sulphonate is absent and the further proviso that the weight of any hydrotrope in the composition is not more than 12% calculated on the weight of the active detergent mixture.

**2.** A composition according to claim 1 wherein at least a major proportion of said component (a) is selected from the group consisting of secondary alkane sulphonate, fatty acyl ester sulphonate, dialkylsulphosuccinate, alpha-olefin sulphonate, primary alkyl sulphate, and mixtures thereof.

**3.** A composition according to claim 1 wherein u, w, x, y and z are given by

$$\frac{u}{22.5} + \frac{w}{30} + \frac{x}{37.5} + \frac{y}{40} + \frac{z}{40} \leqslant 1$$

**4.** A composition according to claim 1 wherein component (a) comprises secondary alkane sulphonate, and component (c) is selected from the group consisting of monoalkanolamides and dialkanolamides,

the active detergent mixture having, in addition to the secondary alkane sulphonate (a) and alkyl sulphate from the alkyl ether sulphate which provides component (b), a maximum content of 5% by weight in total of alkyl sulphate, alkane sulphonate and alkylaryl sulphonate material.

**5.** A composition according to claim 4 wherein the secondary alkane sulphonate is a mixture of materials of different alkyl chain lengths, of the formula

$R_1R_2CHSO_3X$

where $R_1$ and $R_2$ are each a straight or branched chain alkyl group having at least one carbon atom, the alkyl chain length (i.e. total number of carbon atoms of $R_1$ and $R_2$ plus 1) being in the range 13 to 18, and X is a solubilising cation.

**6.** A composition according to claim 1 or claim 3 wherein a major fraction of component (a) is primary alkyl sulphate in an amount which is at least 10% of the active detergent mixture.

**7.** A composition according to any one of the preceding claims wherein the weight ratio of the components (a) and (b) is in the range 1.5:1 to 1:3.

**8.** A composition according to any one of the preceding claims wherein the total amount of alkyl aryl sulphonate, primary alkane sulphonate and alkyl sulphate is not substantially greater than the total amount of components (c) and (d).

**9.** A composition according to any one of the preceding claims wherein the amount of component (a) is not greater than $1\frac{1}{4}$ times the total amount of components (c) and (d).

**10.** A composition according to any one of the preceding claims wherein the total amount of amine oxide and betaine is not more than 20% by weight of the active detergent mixture.

**11.** A composition according to any one of the preceding claims wherein component (c) contains substantially no amine oxide.

**12.** A composition according to any one of the preceding claims wherein component (d) constitutes at least 10% by weight of the active detergent mixture.

**13.** A composition according to any one of the preceding claims wherein each of components (c) and (d) constitutes at least 10% by weight of the active detergent mixture, and the total of components (c) and (d) constitutes at least 25% by weight of the active detergent mixture.

**14.** A composition according to any one of the preceding claims wherein the active detergent mixture has a maximum content of 5% in total of primary alkane sulphonate and alkyl aryl sulphonate.

**15.** A composition according to any one of the preceding claims which is an aqueous liquid composition containing 10 to 40% by weight of the active detergent mixture.

**16.** A detergent composition according to any one of the preceding claims wherein the alkyl ether sulphate (b) is provided by a mixture of materials of the general formula:

$R - (OCH_2CH_2)_n - OSO_3X$

wherein R is a $C_{10}$ to $C_{18}$ alkyl group, X is a solubilising cation, and n, the average degree of ethoxylation, is from 2 to 12.

**17.** A detergent composition according to claim 16 wherein the alkyl ether sulphate (b) is provided by a primary alkyl ether sulphate in which the alkyl groups R are such that less than 20% of material of alkyl chain lengths $C_{14}$ and above is present, and the average degree of ethoxylation n is from 3 to 8.

**18.** A detergent composition according to any one of the preceding claims wherein both monoalkanolamide and dialkanolamide are present, in a weight ratio of monoalkanolamide to dialkanolamide in the range 2:1 to 1:2.

**19.** A detergent composition according to any one of the preceding claims wherein component (d) is selected from the group consisting of polyethoxylated aliphatic alcohols of the general formula:

$R - (OCH_2CH_2)_m - OH$

wherein R is straight or branched alkyl having from 9 to 16 carbon atoms, and the average degree of ethoxylation m is from 5 to 14, and the said

polyethoxylated alkanolamides of the general formula

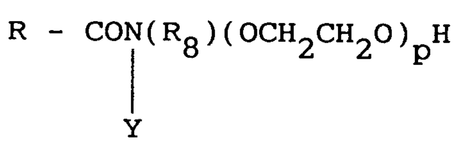

wherein R is a straight or branched alkyl having from 7 to 18 carbon atoms,

$R_8$ is an ethyleneoxy or propyleneoxy group
Y is hydrogen or $-R_8(CH_2CH_2O)_qH$
p is 1 or more and q is 0, 1 or more.

**20.** A detergent composition according to any one of claims 1 to 18 wherein the nonionic component (d) consists essentially only of said polyethoxylated aliphatic alcohol.

**21.** A detergent composition according to claim 20 wherein R in the general formula:

$$R - (OCH_2CH_2)_m - OH$$

is straight or branched alkyl having from 9 to 11 carbon atoms.

**22.** A detergent composition according to claim 18 or claim 21 wherein $\underline{m}$ is from 6 to 12.

**23.** A method of preparing a liquid detergent composition which comprises mixing together materials as set forth in any one of the preceding claims and water, so as to form a composition as set forth in any one of the preceding claims.

**24.** A method of washing articles which comprises diluting a composition according to any one of claims 1 to 22 with water, and washing articles by hand in the resulting solution.

**Patentansprüche**

**1.** Stabiles Reinigungsmittel in flüssiger oder in Gelform, enthaltend von 10 bis 80 Gewichtsprozent einer aktiven Reinigungsmischung und Wasser, wobei die aktive Reinigungsmischung vier Komponenten wie folgt enthält:

(a) Anionisch aktives Detergens, verschieden von Alkylethersulfat, welches in einer Menge im Bereich von 10 bis 60 Gewichtsprozent der aktiven Detergens-Mischung vorhanden ist;

(b) Alkylethersulfat, enthaltend ein oder mehrere Ethylenoxid-Reste pro Molekül, wobei das Gewichtsverhältnis der Komponenten (a) und (b) im Bereich von 2 : 1 bis 1 : 10 liegt, der Anteil der Komponente (b) mit einem einzigen Ethylenoxid-Rest pro Molekül nicht wesentlich größer ist als der Anteil mit der besonders häufig vorkommenden höheren Zahl von Ethylenoxid-Resten pro Molekül;

(c) Schaumverbesserer, ausgewählt aus der Gruppe bestehend aus Monoalkanolamiden, Dialkanolamiden, Betainen, Aminoxiden und Mischungen derselben in einer Gesamtmenge im Bereich von 5 bis 30 Gewichtsprozent der aktiven Reinigungsmischung, wobei die Menge an Monoalkanolamid 20 Gewichtsprozent nicht übersteigt, die Menge an Aminoxid 10 Gewichtsprozent nicht übersteigt, und die Gesamtmenge an Monoalkanolamid, Dialkanolamid und Betain plus dem 1 1/2fachen der Menge des Aminoxids zumindest 7,5 Gewichtsprozent der aktiven Reinigungsmischung beträgt;

(d) nichtionisches aktives Reinigungsmaterial in einer Menge im Bereich von 5 bis 35 Gewichtsprozent der aktiven Reinigungsmischung, wobei das nichtionische aktive Detergens aus der Gruppe bestehend aus polyethoxylierten aliphatischen Alkoholen der allgemeinen Formel

$$R-(OCH_2CH_2)_m-OH$$

besteht, worin R geradkettiges oder verzweigtkettiges Alkyl mit 8 bis 16 Kohlenstoffatomen ist und der durchschnittliche Grad der Ethoxylierung m einen Wert von 5 bis 14 aufweist, und
polyethoxylierte Alkanolamide der allgemeinen Formel

$$R-\underset{\underset{Y}{|}}{CON}(R^8)(OCH_2CH_2O)_pH$$

worin R ein geradkettiges oder verzweigtkettiges Alkyl mit 7 bis 18 Kohlenstoffatomen ist,
$R^8$ eine Ethylenoxy- oder Propylenoxy-Gruppe bedeutet,
Y Wasserstoff oder $-R^8(CH_2CH_2O)_qH$ ist,

p den Wert 1 oder mehr aufweist und q 0, 1 oder mehr ist;

worin die aktive Reinigungsmischung Maximalgehalte u an Alkylarylsulfonat, w an Alkylsulfat, einen maximalen Gesamtgehalt x an sekundärem Alkansulfonat und Fettacylestersulfonat, einen maximalen Gesamtgehalt y an Dialkylsulfosuccinat und $\alpha$-Olefinsulfonat und einen maximalen Gesamtgehalt z von irgendeinem anderen anionischen aktiven Detergens mit Ausnahme des erwähnten Alkylethersulfats hat,

worin u, w, x, y und z alle Gewichtsprozentsätze der reinigungsaktiven Mischung bedeuten und durch

$$\frac{u}{30} + \frac{w}{40} + \frac{x}{50} + \frac{y}{60} + \frac{z}{60} \leqq 1$$

gegeben sind, unter der Bedingung, daß primäres Alkansulfonat abwesend ist und unter der weiteren Bedingung, daß das Gewicht von irgendeinem Hydrotrop in der Zusammensetzung nicht mehr als 12 %, berechnet aufgrund des Gewichts der aktiven Reinigungsmischung, beträgt.

**2.** Mittel nach Anspruch 1, worin zunächst ein Hauptanteil der Komponente (a) aus der Gruppe bestehend aus sekundärem Alkansulfonat, Fettacylestersulfonat, Dialkylsulfosuccinat, $\alpha$-Olefinsulfonat, primärem Alkylsulfat, und Mischungen derselben, ausgewählt ist.

**3.** Mittel nach Anspruch 1, worin u, w, x, y und z durch die nachfolgende Gleichung

$$\frac{u}{22,5} + \frac{w}{30} + \frac{x}{37,5} + \frac{y}{40} + \frac{z}{40} \leqslant 1$$

wiedergegeben werden.

**4.** Mittel nach Anspruch 1, worin die Komponente (a) sekundäres Alkansulfonat enthält und die Komponente (c) aus der Gruppe bestehend aus Monoalkanolamiden und Dialkanolamiden ausgewählt ist, die aktive Reinigungsmischung außer dem sekundären Alkansulfonat (a) und dem Alkylsulfat von dem Alkylethersulfat, welches die Komponente (b) liefert, einen Maximalgehalt von 5 Gewichtsprozent insgesamt an Alkylsulfat, Alkansulfonat und Alkylarylsulfonat-Material enthält.

**5.** Mittel nach Anspruch 4, worin das sekundäre Alkansulfonat eine Mischung von Materialien von verschiedenen Alkylkettenlängen der Formel

$R^1R^2CHSO_3X$

ist, worin jeder der Reste $R^1$ und $R^2$ eine geradkettige oder verzweigtkettige Alkylgruppe mit zumindest einem Kohlenstoffatom bedeutet, die Alkylkettenlänge (d.h. die Gesamtzahl der Kohlenstoffatome von $R^1$ und $R^2$ plus 1) im Bereich von 13 bis 18 ist und X ein solubilisierendes Kation bedeutet.

**6.** Mittel nach Anspruch 1 oder Anspruch 3, worin ein größerer Bruchteil der Komponente (a) primäres Alkylsulfat in einer Menge ist, die zumindest 10 % der aktiven Reinigungsmischung beträgt.

**7.** Mittel nach irgendeinem der vorstehenden Ansprüche, worin das Gewichtsverhältnis der Komponenten (a) und (b) im Bereich von 1,5 : 1 bis 1 : 3 liegt.

**8.** Mittel nach irgendeinem der vorstehenden Ansprüche, worin die Gesamtmenge an Alkylarylsulfonat, primärem Alkansulfonat und Alkylsulfat nicht wesentlich größer ist als die Gesamtmenge der Komponenten (c) und (d).

**9.** Mittel nach irgendeinem der vorstehenden Ansprüche, worin die Menge der Komponente (a) nicht größer als das 1 1/4fache der Gesamtmenge der Komponenten (c) und (d) ist.

**10.** Mittel nach irgendeinem der vorstehenden Ansprüche, worin die Gesamtmenge an Aminoxid und Betain nicht mehr als 20 Gewichtsprozent der aktiven Reinigungsmischung beträgt.

**11.** Mittel nach irgendeinem der vorstehenden Ansprüche, worin die Komponente (c) im wesentlichen kein Aminoxid enthält.

**12.** Mittel nach irgendeinem der vorstehenden Ansprüche, worin die Komponente (d) zumindest 10 Gewichtsprozent der aktiven Reinigungsmischung ausmacht.

**13.** Mittel nach irgendeinem der vorstehenden Ansprüche, worin jede der Komponenten (c) und (d) zumindest 10 Gewichtsprozent der aktiven Reinigungsmischung ausmacht und die Summe der Komponenten (c) und (d) zumindest 25 Gewichtsprozent der aktiven Reinigungsmischung bildet.

**14.** Mittel nach irgendeinem der vorstehenden Ansprüche, worin die aktive Reinigungsmischung einen Maximalgehalt von 5 % insgesamt an primärem Alkansulfonat und Alkylarylsulfonat aufweist.

**15.** Mittel nach irgendeinem der vorstehenden Ansprüche, welches eine wässerige flüssige Zusammensetzung mit einem Gehalt von 10 bis 40 Gewichtsprozent an aktiver Reinigungsmischung ist.

**16.** Reinigungsmittel nach irgendeinem der vorstehenden Ansprüche, worin das Alkylethersulfat (b) durch eine Mischung von Materialien der allgemeinen Formel:

$R\text{-}(OCH_2CH_2)_n\text{-}OSO_3X$

bereitgestellt wird, worin R eine $C_{10-18}$-Alkylgruppe ist, X ein solubilisierendes Kation bedeutet und n, der durchschnittliche Ethoxylierungsgrad, einen Wert von 2 bis 12 aufweist.

**17.** Reinigungsmittel nach Anspruch 16, worin das Alkylethersulfat (b) durch ein primäres Alkylethersulfat bereitgestellt ist, in welchem die Alkylgruppen R so beschaffen sind, daß weniger als 20 % Material von Alkylkettenlängen $C_{14}$ und darüber vorhanden ist, und der durchschnittliche Ethoxylierungsgrad n im Bereich von 3 bis 8 liegt.

**18.** Reinigungsmittel nach irgendeinem der vorstehenden Ansprüche, worin sowohl Monoalkanolamid und Dialkanolamid vorhanden sind, in einem Gewichtsverhältnis von Monoalkanolamid zu Dialkanolamid im Bereich von 2 : 1 bis 1 : 2.

**19.** Reinigungsmittel nach einem der vorhergehenden Ansprüche, worin die Komponente (d) aus der Gruppe bestehend aus polyethoxylierten aliphatischen Alkoholen der allgemeinen Formel:

$$R\text{—}(OCH_2CH_2)_m\text{—}OH$$

besteht, worin R geradkettiges oder verzweigtkettiges Alkyl mit 9 bis 16 Kohlenstoffatomen ist und der durchschnittliche Grad der Ethoxylierung m einen Wert von 5 bis 14 aufweist, und

polyethoxylierte Alkanolamide der allgemeinen Formel

$$R\text{—}\underset{\substack{|\\ Y}}{CON}(R^8)(OCH_2CH_2O)_pH$$

worin R ein geradkettiges oder verzweigtkettiges Alkyl mit 7 bis 18 Kohlenstoffatomen ist,
$R^8$ eine Ethylenoxy- oder Propylenoxy-Gruppe bedeutet,
Y Wasserstoff oder $-R^8(CH_2CH_2O)_qH$ ist,
p den Wert 1 oder mehr aufweist und q 0, 1 oder mehr ist.

**20.** Reinigungsmittel nach irgendeinem der Ansprüche 1 bis 18, worin die nichtionische Komponente (d) im wesentlichen nur aus dem erwähnten polyethoxylierten aliphatischen Alkohol besteht.

**21.** Reinigungsmittel nach Anspruch 20, worin R in der allgemeinen Formel

$$R—(OCH_2CH_2)_m—OH$$

geradkettiges oder verzweigtkettiges Alkyl mit 9 bis 11 Kohlenstoffatomen ist.

**22.** Reinigungsmittel nach Anspruch 18 oder Anspruch 21, worin m einen Wert von 6 bis 12 aufweist.

**23.** Verfahren zur Herstellung eines flüssigen Reinigungsmittels, welches das Zusammenmischen der Materialien, wie sie in irgendeinem der vorstehenden Ansprüche erläutert wurden, und von Wasser umfaßt, um ein Mittel zu bilden, wie es in irgendeinem der vorstehenden Ansprüche erläutert wird.

**24.** Verfahren zum Waschen von Erzeugnissen, welches das Verdünnen einer Zusammensetzung nach irgendeinem der Ansprüche 1 bis 22 mit Wasser und das Waschen der Erzeugnisse mit der resultierenden Lösung, umfaßt.

**Revendications**

**1.** Composition détergente stable sous forme liquide ou de gel contenant de 10 à 80% en poids d'un mélange de détergents actifs et de l'eau, le mélange de détergents actifs comprenant quatre composants comme suit:

(a) un principe actif détergent anionique autre que l'alkyléther sulfate et qui est présent à raison de 10 à 60% en poids du mélange de détergents actifs;

(b) un alkyléther sulfate contenant un ou plusieurs résidus oxyde d'éthylène par molécule, le rapport pondéral des composants (a) et (b) étant compris entre 2:1 et 1:10, la proportion du composant (b) avec un résidu oxyde d'éthylène unique par molécule n'étant pas sensiblement supérieur à la proportion avec le nombre supérieur de répétitions le plus fréquent de résidus oxyde d'éthylène par molécule;

(c) un amplificateur de mousse choisi dans le groupe constitué des monoalcanolamides, des dialcanolamides, des bétaïnes, des oxydes d'amine et de leurs mélanges en une quantité totale comprise entre 5 et 30% en poids du mélange de détergents actifs, la quantité du monoalcanolamide ne dépassant pas 20% en poids, la quantité d'oxyde d'amine ne dépassant pas 10% en poids, et la quantité totale de monoalcanolamide, de dialcanolamide et de bétaïne plus 1 fois et demie la quantité d'oxyde d'amine étant au moins 7,5% en poids du mélange de détergents actifs;

(d) une matière active détergente non ionique à raison de 5 à 35% en poids du mélange de détergents actifs, détergent actif non ionique qui est choisi dans le groupe formé par les alcools aliphatiques polyéthoxylés de formule générale:

$$R - (OCH_2CH_2)_m - OH$$

dans laquelle R est un groupe alkyle ramifié ou droit ayant de 8 à 16 atomes de carbone, et le degré moyen d'éthoxylation m est compris entre 5 et 14, et

les alcanolamides polyéthoxylés de formule générale:

$$R - \underset{\displaystyle Y}{\underset{|}{CON}}(R_8)(OCH_2CH_2O)_pH$$

dans laquelle R est un groupe alkyle ramifié ou droit ayant de 7 à 18 atomes de carbone,

$R_8$ est un groupe éthylèneoxy ou propylèneoxy,

Y est un atome d'hydrogène ou $-R_8(CH_2CH_2O)_pH$

p vaut 1 ou plus et q vaut 0, 1 ou plus;

le mélange de détergents actifs ayant des teneurs maximales u d'alkylaryle sulfonate, w d'alkyle sulfate, une teneur totale maximale x d'alcane secondaire sulfonate et d'ester sulfonate d'acyle gras, une teneur totale maximale y de dialkylsulfosuccinate et d'alpha-oléfine sulfonate et une teneur totale maximale z de tout autre détergent actif anionique, sauf ledit éther sulfate d'alkyle,

où u, w, x, y et z sont tous des pourcentages en poids du mélange du détergent actif et sont donnés dans la relation:

$$\frac{u}{30} + \frac{w}{40} + \frac{x}{50} + \frac{y}{60} + \frac{z}{60} \leq 1$$

à condition que l'alcane primaire sulfonate soit absent et à condition encore que le poids de tout hydrotrope dans la composition ne dépasse pas 12%, calculé sur le poids du mélange de détergents actifs.

**2.** Composition selon la revendication 1, dans laquelle au moins une proportion principale dudit composant (a) est choisie dans le groupe formé par un alcane secondaire sulfonate, un ester d'acyle gras sulfonate, un dialkylsulfosuccinate, un alpha-oléfine sulfonate, un alkyle primaire sulfate, et leurs mélanges.

**3.** Composition selon la revendication 1, dans laquelle u, w, x, y et z sont donnés par

$$\frac{u}{22,5} + \frac{w}{30} + \frac{x}{37,5} + \frac{y}{40} + \frac{z}{40} \leq 1$$

**4.** Composition selon la revendication 1, dans laquelle le composant (a) comprend un alcane secondaire sulfonate, et le composant (c) est choisi dans le groupe formé par les monoalcanolamides et les dialcanolamides,
le mélange de détergents actifs ayant, en plus de l'alcane secondaire sulfonate (a) et de l'alkyle sulfate provenant de l'alkyléther sulfate qui fournit le composant (b), une teneur maximale de 5% en poids du total de la matière d'alkyle sulfate, d'alcane sulfonate et d'alkylaryle sulfonate.

**5.** Composition selon la revendication 4, dans laquelle l'alcane secondaire sulfonate est un mélange de matières de différentes longueurs de chaîne alkyle, de formule

$R_1R_2CHSO_3X$

dans laquelle $R_1$ et $R_2$ représentent chacun un groupe alkyle à chaîne droite ou ramifiée, ayant au moins un atome de carbone, la longueur de chaîne alkyle (à savoir, le nombre total d'atomes de carbone de $R_1$ et $R_2$ plus 1) étant dans la gamme de 13 à 18, et X un cation de solubilisation.

**6.** Composition selon la revendication 1 ou 3, dans laquelle une fraction principale du composant (a) est l'alkyle primaire sulfate en une quantité qui représente au moins 10% du mélange de détergents actifs.

**7.** Composition selon l'une quelconque des revendications précédentes, dans laquelle le rapport pondéral des composants (a) et (b) est compris entre 1,5:1 et 1:3.

**8.** Composition selon l'une quelconque des revendications précédentes, dans laquelle la quantité totale de l'alkylaryl sulfonate, de l'alcane primaire sulfonate et de l'alkyle sulfate n'est pas sensiblement supérieure à la quantité totale des composants (c) et (d).

**9.** Composition selon l'une quelconque des revendications précédentes, dans laquelle la quantité du composant (a) ne dépasse pas 1 fois $\frac{1}{4}$ la quantité totale des composants (c) et (d).

**10.** Composition selon l'une quelconque des revendications précédentes, dans laquelle la quantité totale d'oxyde d'amide et de bétaïne ne dépasse pas 20% en poids du mélange de détergents actifs.

**11.** Composition selon l'une quelconque des revendications précédentes, dans laquelle le composant (c) ne contient sensiblement aucun oxyde d'amine.

**12.** Composition selon l'une quelconque des revendications précédentes, dans laquelle le composant (d) constitue au moins 10% en poids du mélange de détergents actifs.

**13.** Composition selon l'une quelconque des revendications précédentes, dans laquelle chacun des composants (c) et (d) constitue au moins 10% en poids du mélange de détergents actifs, et la totalité des composants (c) et (d) constitue au moins 25% en poids du mélange de détergents actifs.

**14.** Composition selon l'une quelconque des revendications précédentes, dans laquelle le mélange de détergents actifs a une teneur maximale de 5% au total de l'alcane primaire sulfonate et de l'alkylaryle sulfonate.

**15.** Composition selon l'une quelconque des revendications précédentes, qui est une composition liquide aqueuse contenant 10 à 40% en poids du mélange de détergents actifs.

**16.** Composition détergente selon l'une quelconque des revendications précédentes, dans laquelle l'alkyléther sulfate (b) est fourni par un mélange de matières de formule générale:

$R - (OCH_2CH_2)_n - OSO_3X$

dans laquelle R est un groupe alkyle en $C_{10}$ à $C_{18}$, X est un cation de solubilisation, et $\underline{n}$, le degré moyen d'éthoxylation, vaut de 2 à 12.

**17.** Composition détergente selon la revendication 16, dans laquelle l'alkyléther sulfate (b) est fourni par un alkyl(primaire)éthersulfate, dans lequel les groupes alkyle R sont tels que moins de 20% de matière de longueurs de chaîne alkyle en $C_{14}$ et supérieures sont présents, et le degré moyen d'éthoxylation $\underline{n}$ vaut de 3 à 8.

**18.** Composition détergente selon l'une quelconque des revendications précédentes, dans laquelle à la fois le monoalcanolamide et le dialcanolamide sont présents, dans un rapport pondéral de monoalcanolamide au dialcanolamide compris entre 2:1 et 1:2.

**19.** Composition détergente selon l'une quelconque des revendications précédentes, dans laquelle le composant (d) est choisi dans le groupe formé par les alcools aliphatiques polyéthoxylés de formule générale:

$R - (OCH_2CH_2)_m - OH$

dans laquelle R est un groupe alkyle droit ou ramifié ayant de 9 à 6 atomes de carbone, et le degré moyen d'éthoxylation $\underline{m}$ vaut de 5 à 14, et
lesdits alcanolamides polyéthoxylés de formule générale

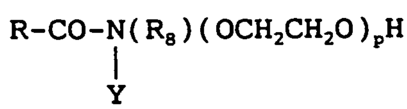

dans laquelle R est un groupe alkyle droit ou ramifié ayant de 7 à 18 atomes de carbone,
$R_8$ est un groupe éthylèneoxy ou propylèneoxy
Y est un atome d'hydrogène ou $-R_8(CH_2CH_2O)_qH$
p vaut 1 ou plus et q vaut 0, 1 ou plus.

**20.** Composition détergente selon l'une quelconque des revendications 1 à 18, dans laquelle le composant anionique (d) ne comprend essentiellement que ledit alcool aliphatique polyéthoxylé.

**21.** Composition détergente selon la revendication 20, dans laquelle R dans la formule générale:

$R - (OCH_2CH_2)_m - OH$

est un groupe alkyle droit ou ramifié, ayant de 9 à 11 atomes de carbone.

**22.** Composition détergente selon la revendication 18 ou 21, dans laquelle m vaut de 6 à 12.

**23.** Procédé pour préparer une composition détergente liquide, qui comprend le mélange conjoint de matières, comme présentées selon l'une quelconque des revendications précédentes et d'eau, afin de former une composition, comme présentée selon l'une quelconque des revendications précédentes.

**24.** Procédé pour laver des articles, qui comprend la dilution d'une composition selon l'une quelconque des revendications 1 à 22 avec de l'eau, et le lavage manuel des articles manuellement dans la solution résultante.